# FASCICULE DE BREVET EUROPEEN

(11) **EP 2 593 437 B1**
(45) Date de publication et mention de la délivrance du brevet: **06.09.2017**
(21) Numéro de dépôt: 11743845.7
(22) Date de dépôt: 12.07.2011
(51) Int. Cl.: C07D 233/36, C08J 3/24, C08F 8/30

(54) **MOLECULES PORTEUSES DE GROUPES ASSOCIATIFS**
MOLEKÜLE MIT KOMBINIERBAREN GRUPPEN
MOLECULES HAVING COMBINABLE GROUPS

(30) Priorité: 13.07.2010 FR 1055717
(43) Date de publication de la demande: 22.05.2013
(73) Titulaire: Arkema France, 92700 Colombes (FR)
(72) Inventeur: SEEBOTH, Nicolas, F-63000 Clermont-Ferrand (FR); IVANOV, Serguey, Orekhovo-Zouevo 142611 (RU); COUTURIER, Jean-Luc, F-69006 Lyon (FR); HIDALGO, Manuel, F-69530 Brignais (FR)
(86) Numéro de dépôt international: PCT/FR2011/051651
(87) Numéro de publication internationale: WO 2012/007684

(56) Documents cités:
- WO-A1-2010/031956
- US-A1- 2004 010 090
- US-B2- 7 186 845
- Simon M. Turega ET AL: "Controlling a recognition-mediated reaction using a pH switch", Chemical Communications, no. 35, 1 January 2006 (2006-01-01), page 3684, XP055125128, ISSN: 1359-7345, DOI: 10.1039/b607551g
- Jean M.J. Tronchet ET AL: "Novel types of cyclonucleosides", Tetrahedron Letters, vol. 31, no. 4, 1 January 1990 (1990-01-01), pages 531-534, XP055125133, ISSN: 0040-4039, DOI: 10.1016/0040-4039(90)87026-V
- ATTANASI O A ET AL: "Synthesis and Reactions of Some Nitrone Derivatives", TETRAHEDRON, ELSEVIER SCIENCE PUBLISHERS, AMSTERDAM, NL, vol. 53, no. 4, 27 January 1997 (1997-01-27), pages 1467-1480, XP004105234, ISSN: 0040-4020, DOI: 10.1016/S0040-4020(96)01081-2

## Description

La présente invention concerne des molécules associatives azotées comprenant au moins un motif les rendant susceptibles de s'associer entre elles ou avec une charge par des liaisons non covalentes, et comprenant une fonction capable de réagir avec un polymère contenant des insaturations pour former une liaison covalente avec ledit polymère.

Dans le domaine industriel, des mélanges de polymères avec des charges sont souvent utilisés. Pour que de tels mélanges présentent de bonnes propriétés, on recherche en permanence des moyens pour améliorer la dispersion des charges au sein des polymères. L'un des moyens pour parvenir à ce résultat est l'utilisation d'agents de couplage capables d'établir des interactions entre le polymère et la charge.

Des agents de couplage d'un polymère avec une charge comprenant des dipôles azotés sont décrits dans les documents publiés sous les numéros US7186845B2 et JP2008208163. WO 2010/031956 décrit aussi des agents de couplage comprenant un reste imidazolidone. Ces documents décrivent la modification de polymères comprenant des motifs diéniques par des composés dipolaires azotés comprenant en outre un hétérocycle, ledit hétérocylce comprenant lui-même un atome d'azote, et un atome d'oxygène et/ou de soufre.

Plus particulièrement les composés décrits sont des nitrones porteuses de fonction oxazolines, thiazolines comme par exemple la (-(2-oxazolyl)- Phenyl-N-methylnitrone)

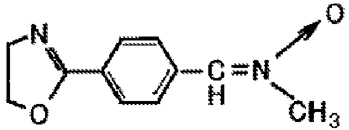

Lorsque des polymères diéniques sont amenés à réagir avec de tels composés, les polymères en résultant porteront les cycles oxazoline ou thiazoline.

Ces cycles présents sur le polymère sont susceptibles de réagir, à leur tour, avec des fonctions de surface des charges (comme le noir de carbone ou la silice) avec lesquels les polymères sont mélangés. Cette réaction conduit à l'établissement de liaisons covalentes entre le polymère modifié par l'agent de couplage et la charge du fait de l'ouverture du cycle oxazoline ou thiazoline. En effet, comme cela est décrit dans le document US7186845B2, les cycles oxazolines et ou thioazolines sont susceptibles de s'ouvrir en présence d'un nucléophile pouvant par exemple être présent à la surface de la charge.

L'établissement de telles liaisons covalentes présente, en revanche, des inconvénients lors de la préparation de mélanges comprenant ces polymères modifiés par des agents de couplage avec des charges. En particulier, l'existence de ces liaisons covalentes précocement établies, entre le polymère et les charges, rend ces mélanges très visqueux à l'état non réticulé ce qui rend difficile toutes les opérations préalables à la réticulation (vulcanisation) des formulations à base de caoutchouc, notamment, la préparation des mélanges des composants, et leur mise en forme ; ces invonvénients ont un fort impact sur la productivité industrielle. Il est donc souhaitable de proposer de nouvelles molécules ne présentant pas les inconvénients ci-dessus, c'est-à-dire des molécules qui soient capables après réaction avec un polymère et mélange avec une charge, de ne pas former de liaisons covalentes avec la charge et donc de ne pas provoquer une trop forte augmentation de la viscosité du mélange.

L'invention concerne un composé comprenant au moins un groupement Q, et au moins un groupement A reliés entre eux par au moins et de préférence un groupement « espaceur » Sp dans lequel :
- Q comprend un dipôle contenant au moins et de préférence un atome d'azote,
- A comprend un groupe associatif comprenant au moins un atome d'azote,
- Sp est un atome ou un groupe d'atomes formant une liaison entre Q et A.

Selon l'invention, Q est un groupe de formule (VII), (VIII) ou (IX) suivante 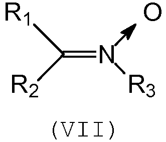 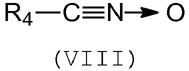 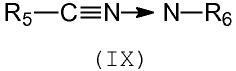 dans lesquelles R1 à R6 sont choisis indépendamment parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 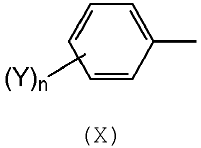 dans laquelle n représente 1, 2, 3, 4 ou 5 et chaque Y représente indépendamment un groupe espaceur Sp, un groupe alkyle ou un halogène

Selon l'invention, A comprend un groupe associatif comprenant au moins un atome d'azote, répondant à la formule (II) suivante : 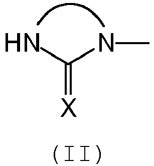 où X désigne un atome d'oxygène ou de soufre,

Selon l'invention, Sp est une chaîne alkyle linéaire ou ramifiée en C1-C24, comprenant éventuellement un ou plusieurs hétéroatomes choisis parmi les atomes d'azote ou d'oxygène.

Un polymère greffé par un composé tel que défini ci-dessus est mélangé à des charges, celui-ci n'établit que des liaisons labiles avec les charges , ce qui permet d'assurer une bonne interaction polymère - charge, bénéfique pour les propriétés finales du polymère, mais sans les inconvénients qu'une trop forte interaction polymère - charge pourrait provoquer.

Les composés objets de l'invention assurent une bonne interaction avec les charges en établissant des liaisons labiles entre les chaînes de polymère et la charge et limitent ainsi les problèmes de mise en oeuvre.

On entend par dipôle une fonction capable de former une addition dipolaire 1,3 sur une liaison carbone-carbone insaturée.

Par « groupe associatif », on entend des groupes susceptibles de s'associer les uns aux autres par des liaisons hydrogène, ioniques et/ou hydrophobes. Il s'agit selon un mode préféré de l'invention de groupes susceptibles de s'associer par des liaisons hydrogène.

Lorsque les groupes associatifs sont susceptibles de s'associer par des liaisons hydrogène, chaque groupe associatif comporte au moins un « site » donneur et un site accepteur vis-à-vis de la liaison hydrogène de sorte que deux groupes associatifs identiques sont auto-complémentaires et peuvent s'associer entre eux en formant au moins deux liaisons hydrogène.

Les groupes associatifs selon l'invention sont également susceptibles de s'associer par des liaisons hydrogène, ioniques et/ou hydrophobes à des fonctions présentes sur des charges.

Les composés selon l'invention comportant un groupement Q, un groupement « espaceur » et un groupement associatif peuvent par exemple être représentés par la formule (Ia) suivante :

A-Sp-Q (Ia).

Les composés selon l'invention comportant un groupement Q, un groupement « espaceur » et deux groupements associatifs peuvent par exemple être représentés par la formule (Ib) suivante :

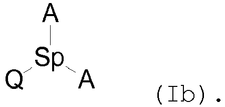

De manière similaire, les composés selon l'invention comportant deux groupements Q, un groupement « espaceur » et un groupement associatif peuvent par exemple être représentés par la formule (Ic) suivante :

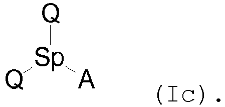

Selon le même principe, les composés selon l'invention comportant deux groupements Q, un groupement « espaceur » et deux groupements associatifs peuvent par exemple être représentés par la formule (Id) suivante :

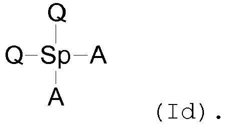

Selon l'invention, le groupement associatif répond à la formule (II) suivante : 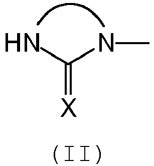 où X désigne un atome d'oxygène ou de soufre, de préférence un atome d'oxygène.

De préférence, le groupement A comprend un hétérocycle di ou triazoté, à 5 ou 6 atomes, de préférence diazoté, et comprenant au moins une fonction carbonyle.

De préférence, le groupement A comprend un groupe imidazolidinyl.

Selon l'invention, le groupement Q est susceptible de se lier à une chaîne polymère comportant au moins une insaturation par liaison covalente (greffage). Tel que défini selon l'invention, le groupement Q comprend une fonction oxyde de nitrile, nitrone, ou nitrile imine pouvant se lier à un polymère porteur d'insaturations, par une cycloaddition de type [3+2].

Le groupement « espaceur » Sp permet de relier au moins un groupement Q et/ou au moins un groupement associatif, A, et ainsi peut être de tout type connu en soi. Le groupement « espaceur » ne doit cependant pas, ou peu, interférer avec les groupements Q et associatif du composé selon l'invention.

Ledit groupement « espaceur » est donc considéré comme un groupement inerte vis-à-vis du groupement Q et du groupement associatif. Par « espaceur » inerte vis-à-vis du groupement Q, on entend : qui ne présente pas de fonctions alcényle ou alcynyle, susceptibles de réagir avec ce groupement. Par « espaceur » inerte vis-à-vis du groupement associatif, on entend : qui ne comprend pas de fonctions associatives telles que définies selon l'invention.

Selon l'invention, le groupement « espaceur » est une chaîne alkyle linéaire ou ramifiée en C1-C24, de préférence C1-C10 éventuellement interrompue par un ou plusieurs atomes d'azote ou d'oxygène, plus préférentiellement une chaîne alkyle linéaire en C1-C6 éventuellement interrompue par un ou plusieurs atomes d'azote ou d'oxygène ;

De préférence le groupement Q est un groupement de formule (XI) : 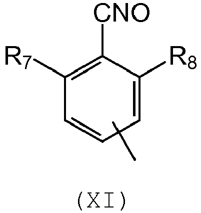 dans laquelle R7 et R8 représentent indépendamment un groupe alkyle en C1-C5 ou un halogène et de préférence R7 et R8 représentent indépendamment un groupe méthyle ou un atome de chlore, et le groupement A est un groupement de formule (XII) :

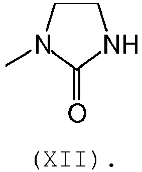

De préférence, le composé objet de l'invention est choisi parmi les composés de formule (XIII) à (XXI) suivantes:

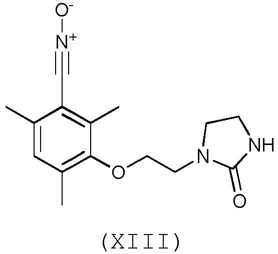

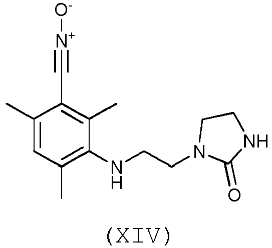

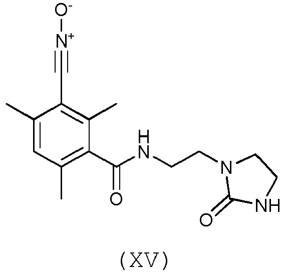

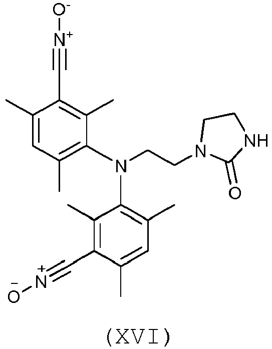

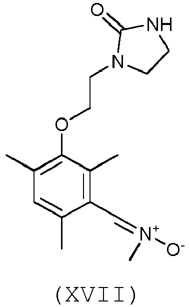

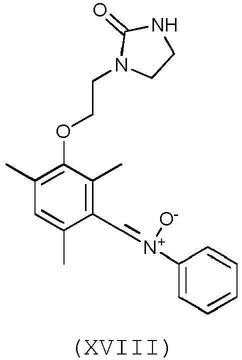

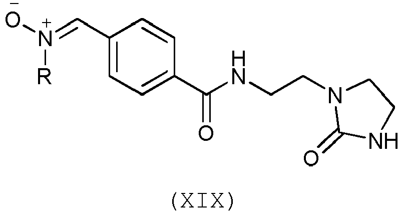

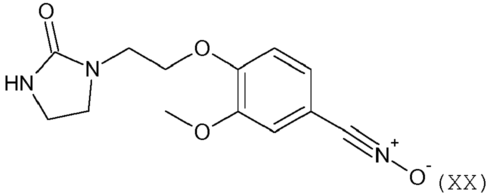

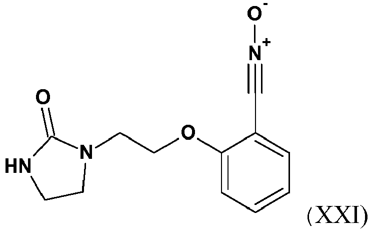

Selon un autre mode de réalisation de l'invention, le composé destiné à greffer le polymère conformément à l'invention est choisi parmi le composé de formule (XXII) à (XXIII) suivantes: 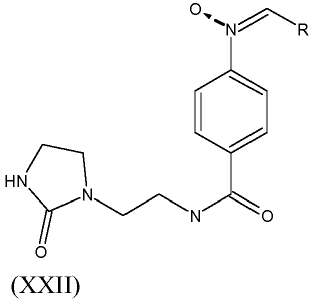 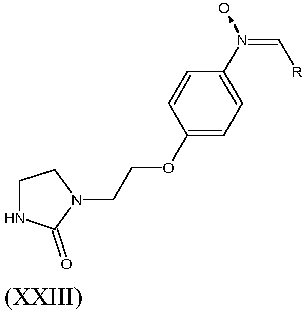 dans lesquelles R est choisi parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 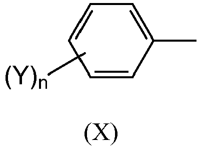 dans laquelle n représente 1, 2, 3, 4 ou 5 et chaque Y représente indépendamment un groupe espaceur Sp, un groupe alkyle ou un halogénure

L'invention est en outre illustrée par les exemples non limitatifs suivants.

### EXEMPLES DE REALISATION

L'analyse structurale ainsi que la détermination des puretés molaires des molécules de synthèses sont réalisées par une analyse RMN. Les spectres sont acquis sur un spectromètre Avance 500 MHz BRUKER équipé d'une sonde " large bande " BBIz-grad 5 mm. L'expérience RMN ¹H quantitative, utilise une séquence simple impulsion 30° et un délai de répétition de 3 secondes entre chacune des 64 acquisitions. Les échantillons sont solubilisés dans le Diméthylsulfoxide deutéré (DMSO). Ce solvant est également utilisé pour le signal de lock. La calibration est réalisée sur le signal des protons du DMSO deutéré à 2.44ppm par rapport à une référence TMS à 0ppm. Le spectre RMN 1H couplé aux expériences 2D HSQC 1H/13C et HMBC 1H/13C permettent la détermination structurale des molécules (cf tableaux d'attributions). Les quantifications molaires sont réalisées à partir du spectre RMN 1D ¹H quantitatif.

La mesure Infrarouge permet de valider la présence du groupement oxyde de nitrile porté par un aromatique. Les spectres sont acquis sur un spectromètre à transformée de Fourier VERTEX 70 équipé d'un détecteur DTGS. Les spectres sont acquis en 32 scans entre 4000cm⁻¹ et 400cm⁻¹ avec une résolution de 2cm⁻¹. Les échantillons sont préparés sous forme de pastilles KBr. La fonction oxyde de nitrile portée par l'aromatique est caractérisée par une bande à 2295cm⁻¹.

L'analyse par spectrométrie de masse est réalisée en injection directe par un mode d'ionisation en electrospray (ID/ESI). Les analyses ont été réalisées sur un spectromètre HCT Bruker (débit 600µL/min, Pression du gaz nébuliseur 10 psi, débit du gaz nébuliseur 4L/min).

### Exemple 1 Préparation du 1-(2-(3'-nitrileoxymesityl-1'-oxy)ethyl)imidazolidin-2-one

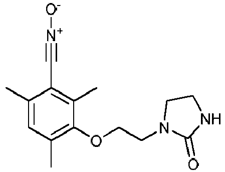

Ce composé peut être préparé à partir du mésitol de l'hydroxyethylimidazolidone selon le schéma synthétique suivant.

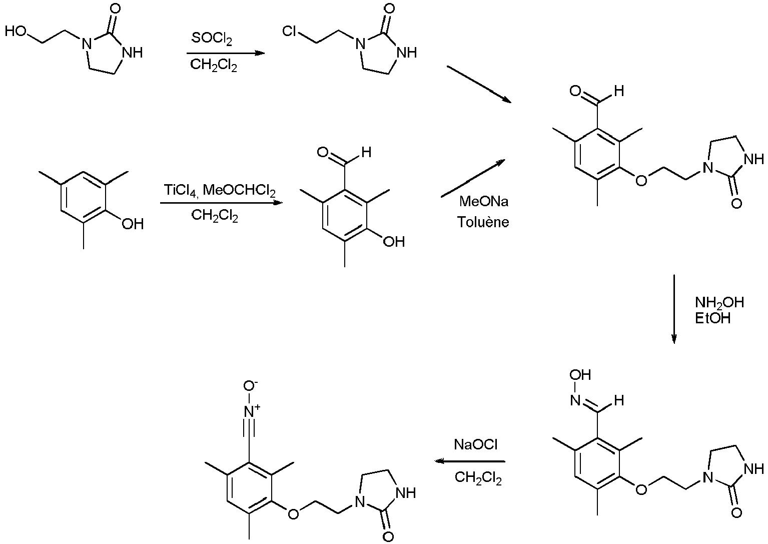

### a) Préparation du 3-hydroxy-2,4,6-trimethylbenzaldehyde

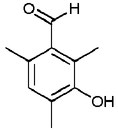

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432*;* Izvestiya Akademii Nauk SSSR, Seriya Khimicheskaya; nb. 7; (1991); p. 1609 - 1615*.*

### b) Préparation de la 1-(2-chloroethyl)imidazolidin-2-one :

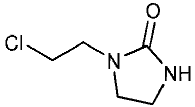

Ce produit est décrit dans l'article Nagarajan K., Arya V. P., Shah R. K.; Indian Journal of Chemistry, Section B: Organic Chemistry Including Medicinal Chemistry; 21; 10; 1982; 928-940*.*

A une solution de 1-(2-hydroxyethyl)imidazolidin-2-one (50,0 g, 0,39 mol) dans le dichlorométhane (250 mL) est ajouté goutte à goutte, à température ambiante, le chlorure de thionyle (34 mL, 0,47 mol) pendant 35 minutes. A la fin de l'addition la température du milieu réactionnel est de 35°C. Le milieu réactionnel est maintenu à une température de 35-40°C pendant 2.5 heures. Après évaporation sous pression réduite (T_{bain} 35 °C, 15-17 mbar) le produit brut est obtenu (67 g). Ce brut est cristallisé dans un mélange d'acétone et d'éther de pétrole (35 g pour 950 mL d'acétone et 820 mL d'éther de pétrole a -24 °C pendant 10 à 15 heures). Les cristaux sont filtrés, lavés par de l'éther de pétrole (2 fois par 40 mL) puis séchés puis pendant 10 à 15 heures sous pression atmosphérique à température ambiante.
Un solide blanc (33,3 g, rendement 66 %) de point de fusion 93 °C est obtenu.
La pureté molaire est supérieure à 97 % (RMN ¹H).

Une caractérisation RMN ¹H et ¹³C est présentée dans le tableau 1 suivant.

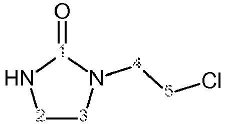

**Tableau 1**

| Atome | δ ¹H (ppm) + mult. | δ ¹³C (ppm) |
|---|---|---|
| 1 | - | 162.1 |
| 2 | 3.17 (t) | 37.5 |
| 3 | 3.33 (t) | 44.7 |
| 4 | 3.29 (t) | 45.0 |
| 5 | 3.62 (t) | 42.4 |

Solvant utilisé : DMSO - Calibration sur le signal du DMSO à 2.44 ppm en ¹H, 39.5 ppm en ¹³C.

### c) Préparation de 2,4,6-trimethyl-3-(2-(2-oxoimidazolidin-1-yl)ethoxy)benzaldehyde :

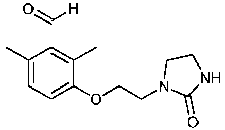

A une solution de sodium (1.63 g, 0.071 mol.) dans le méthanol (60 mL) est ajouté goutte à goutte le 3-hydroxy-2,4,6-trimethylbenzaldehyde (11.90 g, 0.073 mol.) dans le toluène anhydre (300 mL). Le mélange est porté au reflux puis le méthanol est distillé (volume de mélange azéotropique recueilli 80-90 mL). Après retour à 80-90 °C, la (2-chloroethyl)imidazolidin-2-one (10.45 g, 0,070 mol) est ajoutée en une fois au milieu réactionnel. Après 7 heures de reflux, les solvants sont évaporés sous pression réduite (T_{bain} 50 °C, 25 mbar). Au mélange obtenu est ajoutée du dichloromethane (150 mL) et de l'eau (30 mL). La phase organique est lavée ensuite 2 fois à l'eau (20 mL). Après séchage sur Na₂SO₄, le dichlorométhane est évaporé sous pression réduite (T_{bain} 35 °C, 33 mbar). Au mélange obtenu (24 g) est ajoutée de l'éther de pétrole (3 fois par 50 mL) et de l'eau (50 mL) et le précipite obtenu est filtré et lavé sur le filtre par de l'eau (15 mL) et de l'éther de pétrole (2 fois par 15 mL).
Le produit obtenu est repurifié par un lavage du produit en solution dans le dichlorométhane (80 mL) par une solution de NaOH à 4% dans l'eau (3 fois par 60 mL). Après évaporation des solvants sous pression réduite, le produit est précipité dans de l'éther de pétrole. Le précipité est filtré et séché pendant 15 à 20 heures sous pression atmosphérique a température ambiante.
Un solide blanc (8,55 g, rendement 44 %) de point de fusion 139 °C est obtenu.
La pureté molaire est supérieure à 94 % (RMN 1H).

Une caractérisation RMN ¹H et ¹³C est présentée dans le tableau 2 suivant.

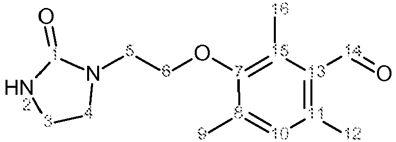

**Tableau 2**

| Atome | δ ¹H (ppm) + mult. | δ ¹³C (ppm) |
|---|---|---|
| 1 | - | 163.1 |
| 2 | ∼ 4.74 (s) | - |
| 3 | 3.40 (t) | 38.1 |
| 4 | 3.65 (t) | 46.8 |
| 5 | 3.52 (t) | 43.9 |
| 6 | 3.79 (t) | 71.3 |
| 7 | - | 153.9 |
| 8 | - | * |
| 9 | 2.23/2.46 (s) | 16.5/19.8 |
| 10 | 6.84 | 131.7 |
| 11 | - | * |
| 12 | 2.23/2.46 (s) | 16.5/19.8 |
| 13 | - | * |
| 14 | ∼ 10.46 (s) | 193.0 |
| 15 | - | * |
| 16 | 2.46 (s) | 12.1 |

| | | |
|---|---|---|
| * 131.4/133.5/136.6/136.7 ppm : Les déplacements chimiques des ¹³C du cycle aromatique ne sont pas attribués. | | |

Solvant utilisé : CDCl₃ - Calibration sur le signal du chloroforme à 7.2 ppm en ¹H, 77 ppm en ¹³C.

### d) Préparation du 2,4,6-trimethyl-3-(2-(2-oxoimidazolidin-1-yl)ethoxy)benzaldehyde oxime :

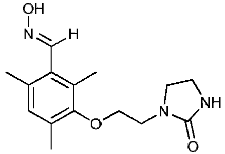

A une solution de 2,4,6-trimethyl-3-(2-(2-oxoimidazolidin-1-yl)ethoxy)benzaldehyde (7,90 g, 0,029 mol) dans l'éthanol (70 mL) maintenue à une température de 45°C est ajoutée une solution d'hydroxylamine aqueuse (2,83 g, 0,043 mol., 50 % dans l'eau) dans l'éthanol (10 mL). Le milieu réactionnel est ensuite agité pendant 2.5 heures à une température comprise entre 50 et 55°C. Le solvant est évaporé sous pression réduite (T_{bain} 37 °C, 35 mbar). Au brut obtenu est ajouté de l'éther de pétrole (80 mL). Le précipité obtenu est filtré, lavé par de l'éther de pétrole (2 fois par 20 mL) et séché pendant 15 à 20 heures sous pression atmosphérique à température ambiante.

Un solide blanc (7,82 g, rendement 94 %) de point de fusion 165 °C est obtenu.

La pureté molaire est supérieure à 84 % (les 16 % restants comprennent notamment 7 % mol. d'EtOH) d'après la RMN ¹H.

Une caractérisation RMN ¹H et ¹³C est présentée dans le tableau 3 suivant.

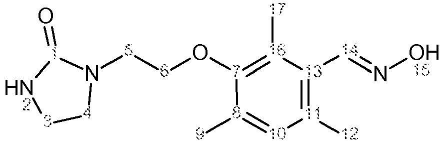

**Tableau 3**

| Atome | δ ¹H (ppm) + mult. | δ ¹³C (ppm) |
|---|---|---|
| 1 | - | 162.0 |
| 2 | ∼ 6.30 (s) | - |
| 3 | 3.19 (t) | 37.1 |
| 4 | 3.44 (t) | 45.5 |
| 5 | 3.34 (t) | 43.2 |
| 6 | 3.69 (t) | 70.3 |
| 7 | - | 153.5 |
| 8 | - | * |
| 9 | 2.14 (s) | 15.4 |
| 10 | - | 130.5 |
| 11 | - | * |
| 12 | 2.18 (s) | 19.9 |
| 13 | - | * |
| 14 | ∼ 8.20 (s) | 147.4 |
| 15 | ∼ 11.10 (s) | - |
| 16 | - | * |
| 17 | 2.17 (s) | 12.9 |

| | | |
|---|---|---|
| * 129.3/129.5/131.9 ppm : Les déplacements chimiques des ¹³C du cycle aromatique ne sont pas attribués, trois signaux sont détectés (probablement deux carbones sous un même signal). | | |

Solvant utilisé : DMSO - Calibration sur le signal du DMSO à 2.44 ppm en ¹H, 39.5 ppm en ¹³C.

### e) Préparation de 2,4,6-trimethyl-3-(2-(2-oxoimidazolidin-1-yl)ethoxy) nitriloxide, composé selon l'invention :

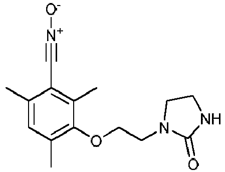

A une solution d'oxime précédemment préparée (6,00 g, 0,021 mol) dans le dichlorométhane (250 mL), à la température de 2°C, est ajoutée goutte à goutte une solution aqueuse de NaOCl (4 % du chlore actif, 52 mL) pendant 5-7 minutes. La température du milieu réactionnel est maintenue entre 0 et - 4°C. Le milieu réactionnel est ensuite agité pendant 3 heures à une température comprise entre 0 et 5°C. La phase organique est alors séparée. La phase aqueuse est extraite par du dichlorométhane (2 fois par 15 mL). Les phases organiques sont rassemblées puis lavées à l'eau (2 fois par 20 mL), séchées par Na₂SO₄. Le volume de solvant est réduit par évaporation sous pression réduite (T_{bain} 22 °C, 220 mbar) jusqu'à 50-60 mL. De l'éther de pétrole (75 mL) est alors ajouté et la solution est placée à -18°C pendant 10-15 heures. Le précipité obtenu est filtré et lavé par un mélange acétate d'éthyle / éther de pétrole (1/2) (10 mL) et enfin séché pendant 10-15 heures sous pression atmosphérique à température ambiante.
Un solide blanc (4,70 g, rendement 79 %) de point de fusion 156 °C est obtenu.
La pureté molaire est supérieure à 85 % (RMN ¹H).

Une caractérisation RMN ¹H et ¹³C est présentée dans le tableau 4 suivant.

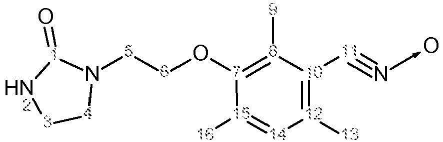

**Tableau 4**

| Atome | δ ¹H (ppm) + mult. | δ ¹³C (ppm) |
|---|---|---|
| 1 | - | Non détecté, non attribué |
| 2 | ∼ 4.59 (s) | - |
| 3 | 3.41 (t) | 38.3 |
| 4 | 3.64 (t) | 47.0 |
| 5 | 3.51 (t) | 44.1 |
| 6 | 3.79 (t) | 71.5 |
| 7 | - | 153.6 |
| 8 | - | 134.4/137.3* |
| 9 | 2.32 (s) | 14.8 |
| 10 | - | 112.8 |
| 11 | - | Non détecté, non attribué |
| 12 | - | 134.4/137.3* |
| 13 | 2.31 (s) | 20.2 |
| 14 | 6.85 (s) | 130.3 |
| 15 | - | 134.4/137.3* |
| 16 | 2.20 (s) | 16.4 |

| | | |
|---|---|---|
| * Les carbones aromatiques 8, 12 et 15 ne sont pas attribués. On observe deux signaux en RMN ¹³C, on a probablement deux carbones qui sortent sous le même signal. | | |

La fonction -C≡N→O présente une bande IR caractéristique à 2295 cm⁻¹

Solvant utilisé : CDCl₃ - Calibration sur le signal du chloroforme à 7.2 ppm en ¹H, 77 ppm en ¹³C.

### Exemple 2 Préparation du 2-[2-(2-oxoimidazolidin-1-yl)ethoxy]benzonitrile oxide

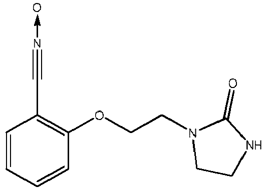

Ce composé peut être préparé à partir de l'aldéhyde salicylique et de la 2-chloroethylimidazolidone selon le schéma synthétique suivant :

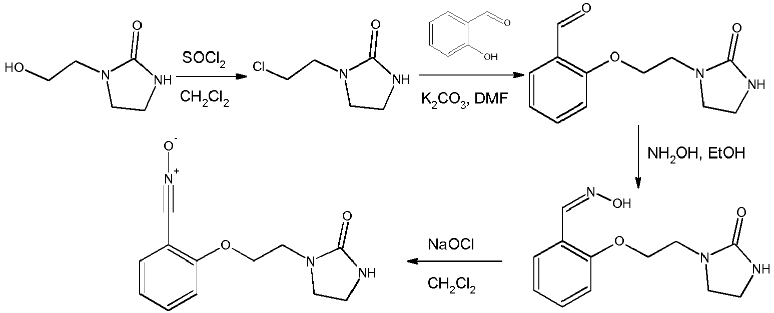

### a) La préparation de la 1-(2-chloroethyl)imidazolidin-2-one est décrite dans l'exemple 1.

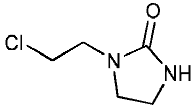

### b) Préparation du 2-[2-(2-oxoimidazolidin-1-yl)ethoxy]benzaldehyde

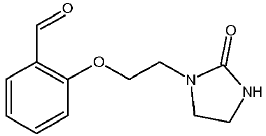

A une solution d'aldéhyde salicylique (22.0g, 0.180 mol) dans le DMF (100 mL) est ajouté du K₂CO₃ (87.1g, 0.631 mol). Le mélange est agité à 52°C. Après 10 minutes à cette température, est ajouté par portions le 1-(2-chloroethyl)imidazolidin-2-one (40,0 g, 0,270 mol, pureté > 90 %). La température du mélange est amenée à 90°C (T_{bain}) en une heure et cette température est maintenue pendant 5 heures. Après retour à température ambiante, le mélange est dilué par de l'eau (1,3 L) et le produit est extrait par CH₂Cl₂ (500 mL, 5 fois 100 mL). Les phases organiques sont rassemblées, puis lavées par l'eau (deux fois par 50 mL) et évaporées jusqu'à obtention d'un brut réactionnel de 70-80 g (suspension dense) (T_{bain} = 40°C). Le brut réactionnel est repris dans Et₂O (120 mL) et la suspension est agitée à température ambiante pendant 20 minutes. Le précipité obtenu est filtré et lavé par un mélange de DMF/Et₂O/H₂O (5mL/20mL/15mL) puis par Et₂O (2 fois par 10 mL). Le solide obtenu est séché à température ambiante.

Un solide (30,6 g, rendement 73 %) de point de fusion 150 °C est obtenu. La pureté molaire est supérieure à 84 % (RMN ¹H).

Le 2-[2-(2-oxoimidazolidin-1-yl)ethoxy]benzaldehyde obtenu est directement engagé dans l'étape suivante sans purification supplémentaire.

### Caractérisation RMN ¹H et ¹³C

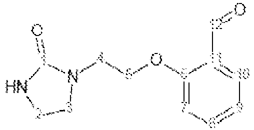

**Tableau 5**

| Atome | δ ¹H (ppm) | δ ¹³C (ppm) |
|---|---|---|
| 1 | - | 164.9 |
| 2 | 3.15 | 37.3 |
| 3 | 3.39 | 44.9 |
| 4 | 3.44 | 42.1 |
| 5 | 4.16 | 66.5 |
| 6 | - | 160.5 |
| 7 | 7.17 | 113.2 |
| 8 | 7.59 | 136.2 |
| 9 | 7.02 | 120.5 |
| 10 | 7.63 | 127.3 |
| 11 | - | 124.0 |
| 12 | 10.31 | 189.1 |

Solvant utilisé : DMSO - Calibration sur le signal du DMSO à 2.44 ppm en ¹H, 39.5 ppm en ¹³C.

### c) Préparation du 2-[2-(2-oxoimidazolidin-1-yl)ethoxy]benzaldehyde oxime

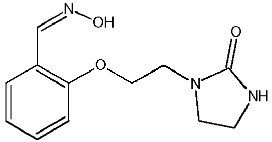

Une solution du 2-[2-(2-oxoimidazolidin-1-yl)ethoxy]benzaldehyde (10,0 g, 0,043 mol) dans EtOH (100 mL) est portée à 50°C. A cette température, une solution d'hydroxylamine (4,5 g, 0,068 mol, 50 % dans l'eau, Aldrich) dans EtOH (10 mL) est ajoutée. Le milieu réactionnel est ensuite agité pendant 6 heures à une température comprise entre 50°C et 70°C. Le milieu réactionnel est évaporé sous pression réduite (T_{bain} 45 °C, 65-70 mbar) jusqu'à l'obtention d'une suspension. Le brut réactionnel est ensuite repris dans l'eau (5 mL). La solution obtenue est refroidie à 5°C et maintenue à cette température pendant 15 heures. Le précipité obtenu est filtré et lavé sur le filtre par un coupage EtOH/eau (2 mL/2 mL) puis par un coupage EtOH/éther de pétrole (1 mL / 4 mL) puis par de l'éther de pétrole (2*10 mL). Le solide est alors séché sous pression atmosphérique à température ambiante.

Un solide blanc (9,25 g, rendement 87 %) de point de fusion 88 °C est obtenu.

La pureté molaire est supérieure à 99 % (RMN ¹H).

### Caractérisation RMN ¹H et ¹³C

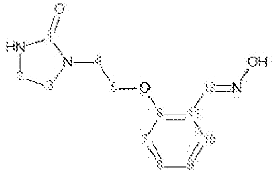

**Tableau 6**

| Atome | δ ¹H (ppm) | δ ¹³C (ppm) |
|---|---|---|
| 1 | - | 162.0 |
| 2 | 3.17 | 37.4 |
| 3 | 3.37 | 45.4 |
| 4 | 3.39 | 42.6 |
| 5 | 4.03 | 66.6 |
| 6 | - | 155.8 |
| 7 | 7.01 | 113.0 |
| 8 | 7.28 | 130.7 |
| 9 | 6.89 | 121.2 |
| 10 | 7.61 | 125.2 |
| 11 | - | 120.9 |
| 12 | 8.25 | 143.3 |

Solvant utilisé : DMSO - Calibration sur le signal du DMSO à 2.44 ppm en ¹H, 39.5 ppm en ¹³C.

### d) Préparation du 2-[2-(2-oxoimidazolidin-1-yl)ethoxy]benzonitrile oxide

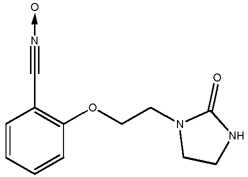

A une suspension de 2-[2-(2-oxoimidazolidin-1-yl)ethoxy]benzaldehyde oxime (20,2 g, 0,081 mol) dans CH₂Cl₂ (400 mL) à -1°C est ajoutée, goutte à goutte pendant 10 minutes, une solution aqueuse de NaOCl dans l'eau (157 mL, Aldrich, > 4 % de chlore actif). Le milieu réactionnel est ensuite agité pendant 20 minutes. Les phases aqueuses et organiques sont séparées et la phase aqueuse est extraite par CH₂Cl₂ (2 fois 75 mL). Les phases organiques rassemblées sont lavées par l'eau (3 fois 10 mL) et séchées sur Na₂SO₄. Les phases sont concentrées à 100mL sous pression réduite à température ambiante. 50 mL d'éther de pétrole sont ajoutés. La solution est refroidie jusqu'à -18°C (3 heures). Le précipité est filtré, lavé par CH₂Cl₂ / éther de pétrole (5 mL/ 10 mL ; puis 5 mL/ 20 mL; puis 0 mL/ 20 mL) puis séché sous pression atmosphérique à température ambiante.

Un solide (11,32 g, rendement 57 %) de point de fusion 109-110 °C avec dégradation du produit est obtenu.
La pureté molaire est supérieure à 94 % (RMN ¹H).

### Caractérisation RMN ¹H et ¹³C

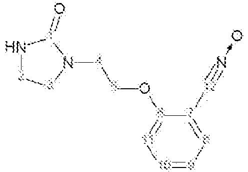

**Tableau 7**

| Atome | δ ¹H (ppm) | δ ¹³C (ppm) |
|---|---|---|
| 1 | - | 162.0 |
| 2 | 3.18 | 37.5 |
| 3 | 3.45 | 45.8 |
| 4 | 3.39 | 42.5 |
| 5 | 4.14 | 67.9 |
| 6 | - | 159.9 |
| 7 | - | 101.6 |
| 8 | 7.60 | 133.4 |
| 9 | 7.00 | 121.2 |
| 10 | 7.48 | 132.9 |
| 11 | 7.16 | 112.6 |
| 12 | - | Non observé |
| NH | ∼ 6.34 | - |

Solvant utilisé : DMSO - **Calibration** sur le signal du DMSO à 2.44 ppm en ¹H, 39.5 ppm en ¹³C.

### Caractérisation Infra-Rouge (pastille KBr)

*v* (cm⁻¹) : 2295 (fonction Ar-C≡N**→**O)

### Caractérisation par spectrométrie de masse

**C**₁₂**H**₁₃**N**₃**O**₃, **Mw= 247.25 g/mol**

Les échantillons ont été analysés par introduction directe dans le spectromètre de masse, en utilisant le mode d'ionisation par électrospray (ID/ESI).

### Préparation de l'échantillon

20 mg de l'échantillon est dissous dans 2 mL d'acétonitrile

***m*/*z* :** 270 ([[M+Na]⁺), 517 ([2M+Na]⁺)

### Exemple 3 : Préparation du 3-methoxy-4-[2-(2-oxoimidazolidin-1-yl)ethoxy]benzonitrile oxide

Ce composé peut être préparé à partir de la vanilline et de la 2-chloroethylimidazolidone selon le schéma synthétique suivant :

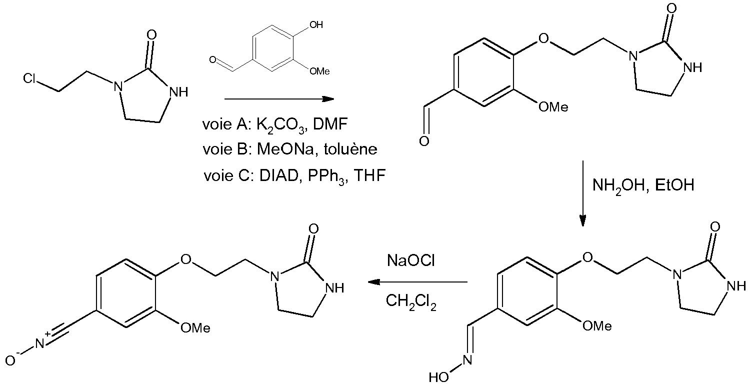

### a) Préparation du 3-methoxy-4-[2-(2-oxoimidazolidin-1-yl)ethoxy]benzaldehyde

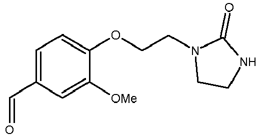

### Voie A

Une suspension de vanilline (30,0 g, 0,197 mol) et de K₂CO₃ (95,4 g, 0,690 mol) dans le DMF (200 mL) est portée à 50°C pendant 15 minutes. A cette suspension est ajouté par portions le 1-(2-chloroethyl)imidazolidin-2-one (44,0 g, 0,296 mol, pureté > 90%) dans le DMF (30 mL). Le milieu réactionnel est chauffé à 90°C (T_{bain}) et cette température est maintenue pendant environ 4 heures. Le milieu réactionnel est ramené à température ambiante puis de l'eau (1,25 L) est ajoutée. Le produit est extrait par CH₂Cl₂ (400 mL, 4 fois par 100 mL). Les phases organiques rassemblées sont lavées par l'eau (60 mL) et concentrées sous pression réduite (14 mbar, 40°C). Le brut réactionnel est dilué par Et₂O (100 mL) et la suspension est agitée à température ambiante pendant 15-20 minutes. Le précipite obtenu est filtré, lavé par Et₂O (3 fois par 15 mL) et séché à température ambiante.

Un solide (31.2 g, rendement 60 %) de point de fusion 130 °C est obtenu.

La pureté molaire est supérieure à 92 % (RMN ¹H).

### Voie B

A une solution de sodium (1,51 g, 0,066 mol) dans CH₃OH (60 mL) est ajoutée de la vanilline (10,0 g, 0,066 mol) dans le toluène anhydre (250 mL). Le milieu réactionnel, sous atmosphère inerte, est porté au reflux puis le méthanol résiduel est distillé. Après retour à 80-90 °C, une suspension de 1-(2-chloroethyl)imidazolidin-2-one (9,28 g, 0,064 mol, pureté > 95%) dans le toluène (50 mL) est ajoutée en une fois au milieu réactionnel. Après 25 heures de réaction, le milieu réactionnel est concentré sous pression réduite (T_{bain} 50°C, 30 mbar). Le brut réactionnel est repris dans CH₂Cl₂ (150 mL). La vanilline non réagie est éliminée par extraction avec une solution aqueuse de 7 % NaOH (5 fois par 30 mL). Les phases organiques rassemblées sont lavées par de l'eau (4 fois 50 mL), séchées sous Na₂SO₄ et évaporées sous pression réduite (T_{bain} 27 °C, 20 mbar). Le brut réactionnel (4,81 g) est dilué par un mélange d'éther de pétrole et EtOAc et le précipité obtenu est filtré.

Un solide (0,91 g, rendement 6 %) de point de fusion 127 °C est obtenu.

La pureté molaire est supérieure à 81 % (RMN ¹H).

### Voie C

Le mode opératoire de la réaction de Mitsunobu est par exemple décrit dans les références suivantes : Mitsunobu, O.; Yamada, Y. Bull. Chem. Soc. Japan 1967, 40, 2380-2382, The Use of Diethyl Azodicarboxylate and Triphenylphosphine in Synthesis and Transformation of Natural Products Mitsunobu, O. Synthesis 1981, 1-28, brevet EP1149092 B1, 2003.

A une solution de vanilline (5,02 g, 0,033 mol), de 1-(2-hydroxyethyl)imidazolidin-2-one anhydre (6,38 g, 0,049 mol, Aldrich) et de PPh₃ (13,1 g, 0,050 mol) dans le THF anhydre (300 mL) à 2°C est ajouté goutte à goutte, sur 20 minutes, une solution de diisopropyl azodicarboxylate (10,1 g, 0,050 mol, Aldrich) dans le THF anhydre (150 mL). Le milieu réactionnel est agité pendant 14 heures à température ambiante puis est dilué par l'eau (150 mL). Le milieu réactionnel est concentré sous pression réduite (45 mbar, T_{bain} 28°C). La phase aqueuse est extraite par EtOAc (3 fois par 200 mL). Les phases organiques rassemblées sont lavées par une solution aqueuse de NaCl saturée puis sont concentrées sous pression réduite de façon à obtenir une solution de 150 mL. Le brut réactionnel en solution est purifié par chromatographie sur colonne (SiO₂, éluant 1 : EtOAc, éluant 2 : EtOAc/EtOH = 4/1, Rf du produit 0,36, Rf de Ph₃PO 0,71 dans EtOAc : EtOH = 5:1).

Un solide (6,59 g, rendement 76 %) de point de fusion 130 °C est obtenu.

La pureté molaire est supérieure à 88 % (RMN ¹H).

Le 3-methoxy-4-[2-(2-oxoimidazolidin-1-yl)ethoxy]benzaldehyde obtenu est directement engagé dans l'étape suivante sans purification supplémentaire.

### Caractérisation RMN ¹H et ¹³C

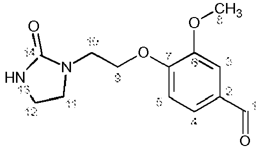

**Tableau 8**

| **N° atomes** | **δ du ¹H (ppm)** | **δ du ¹³C (ppm)** |
|---|---|---|
| 1 | 9,78 | 191,1 |
| 2 | / | 129,6 |
| 3 | 7,34 | 109,6 |
| 4 | 7,48 | 125,6 |
| 5 | 7,14 | 112,0 |
| 6 | / | 148,9 |
| 7 | / | 152,9 |
| 8 | 3,78 | 55,4 |
| 9 | 4,11 | 67,3 |
| 10 | 3,42 | 45,5 |
| 11 | 3,38 | 42,3 |
| 12 | 3,16 | 37,2 |
| 13 | 6,33 | / |
| 14 | / | 161,9 |

Solvant utilisé : DMSO - Calibration sur le signal du DMSO à 2.44 ppm en ¹H, 39.5 ppm en ¹³C.

### b) Préparation 3-methoxy-4-[2-(2-oxoimidazolidin-1-yl)ethoxy]benzaldehyde oxime

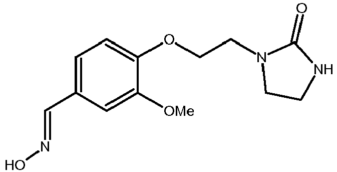

A une solution de 3-methoxy-4-[2-(2-oxoimidazolidin-1-yl)ethoxy]benzaldehyde (25,6 g, 0,097 mol) dans EtOH (250 mL) à 52 °C est ajoutée une solution d'hydroxylamine (10,2 g, 0,155 mol, 50 % dans l'eau, Aldrich) dans EtOH (20 mL). Le milieu réactionnel est ensuite agité pendant 4,5 heures entre 50 et 60°C. Le milieu réactionnel est ensuite concentré sous pression réduite (T_{bain} = 42 °C, 60 mbar) de façon à obtenir un résidu de 70-80 mL. Le précipité obtenu est filtré, lavé par un mélange EtOH/eau (2 fois 5 mL/15 mL) et séché sous pression atmosphérique à température ambiante.

Un solide blanc (22,14 g, rendement 82 %) de point de fusion 189 °C est obtenu.

La pureté molaire est supérieure à 89 % (RMN ¹H).

### Caractérisation RMN ¹H et ¹³C

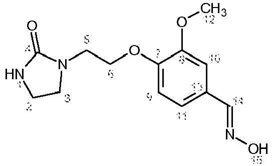

**Tableau 9**

| **N° atomes** | **δ du ¹H (ppm)** | **δ du ¹³C (ppm)** |
|---|---|---|
| 1 | 6,30 | / |
| 2 | 3,16 | 37,1 |
| 3 | 3,35 | 42,4 |
| 4 | / | 161,9 |
| 5 | 3,42 | 45,4 |
| 6 | 4,00 | 67,0 |
| 7 | / | 148,5 |
| 8 | / | 148,9 |
| 9 | 6,93 | 112,8 |
| 10 | 7,15 | 108,6 |
| 11 | 7,01 | 119,9 |
| 12 | 3,72 | 55,2 |
| 13 | / | 125,9 |
| 14 | 7,98 | 147,5 |
| 15 | 10,92 | / |

Solvant utilisé : DMSO - Calibration sur le signal du DMSO à 2.44 ppm en ¹H, 39.5 ppm en ¹³C.

### c) Préparation du 3-methoxy-4-[2-(2-oxoimidazolidin-1-yl)ethoxy]benzonitrile oxide

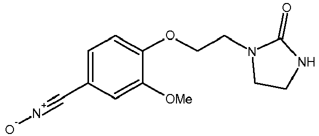

A une suspension de 3-methoxy-4-[2-(2-oxoimidazolidin-1-yl)ethoxy]benzaldehyde oxime (21,7 g, 0,078 mol) dans CH₂Cl₂ (950 mL) à -3°C, est ajoutée, goutte à goutte, une solution aqueuse de NaOCl dans l'eau (Aldrich, > 4 % de chlore actif) (161 mL) pendant 10 minutes. Le milieu réactionnel est ensuite agité 20 minutes à 0°C. La phase organique est séparée et la phase aqueuse est extraite par CH₂Cl₂ (4 fois par 100 mL). Les phases organiques rassemblées sont lavées par l'eau (3 fois par 100 mL), séchées sur Na₂SO₄ puis concentrées sous pression réduite (T_{bain} 22°C) jusqu'à 200-220 mL. Le précipité obtenu est filtré, lavé par CH₂Cl₂ (2 fois par 10 mL) et séché sous pression atmosphérique à température ambiante.

Un solide (9,13 g, rendement 42 %) de point de fusion 109-111 °C avec dégradation est obtenu.

La pureté molaire est supérieure à 80 % (RMN ¹H). Par recristallisation dans EtOH, la pureté du composé est supérieure à 90% massique

### Caractérisation RMN ¹H et ¹³C

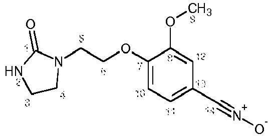

**Tableau 10**

| **N° atomes** | **δ du** ¹**H (ppm)** | **δ du** ¹³**C (ppm)** |
|---|---|---|
| 1 | / | 163,5 |
| 2 | 6,31 | / |
| 3 | 3,15 | 37,2 |
| 4 | 3,35 | 42,3 |
| 5 | 3,40 | 45,4 |
| 6 | 4,04 | 67,1 |
| 7 | / | 150,4 |
| 8 | / | 148,4 |
| 9 | 3,73 | 55,6 |
| 10 | 7,03 | 113,0 |
| 11 | 7,25 | 125,8 |
| 12 | 7,32 | 115,2 |
| 13 | / | 106,2 |
| 14 | / | Non visible |

Solvant utilisé : DMSO - Calibration sur le signal du DMSO à 2.44 ppm en ¹H, 39.5 ppm en ¹³C.

### Caractérisation Infra-Rouge (pastille KBr)

*v* (cm⁻¹) : 2305 (fonction Ar-C≡N**→**O)

### Caractérisation par spectrométrie de masse

**C**₁₃**H**₁₅**N**₃**O**₄**, Mw= 277.27 g/mol**

Les échantillons ont été analysés par introduction directe dans le spectromètre de masse, en utilisant le mode d'ionisation par électrospray (ID/ESI).

### Préparation de l'échantillon

Environ 20 mg d'échantillon a été mis en solution dans 25 ml de méthanol puis dilué au 1/100 pour l'analyse par ID/ESI.

### Mode positif :

***m*/*z* :** 300 ([[M+Na]⁺), 577 ([2M+Na]⁺)

### Exemple 4: Préparation de la (Z,E)-N-(4-(2-(2-oxoimidazolidin-1-yl)ethylcarbamoyl)benzylidene)aniline oxide

Ce composé peut être préparé à partir de l'acide 4-formylbenzoïque et de la 2-aminoethylimidazolidone selon le schéma synthétique suivant :

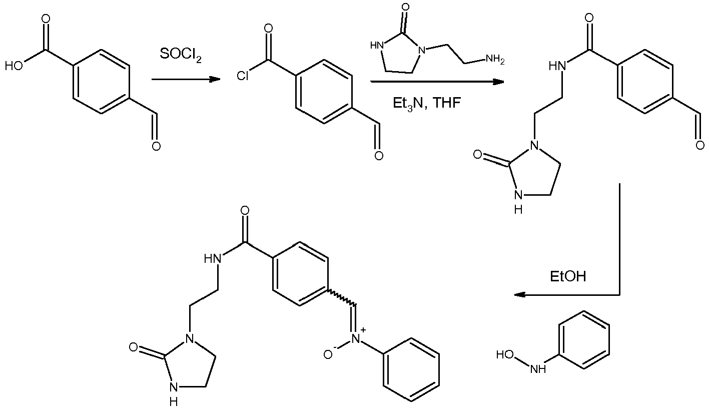

### a) Préparation du Chlorure de 4-formylbenzoyle

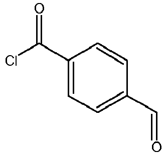

La synthèse de ce composé est décrite dans les références suivantes : **JANSSEN PHARMACEUTICA N.V.;** WO2007/53386; (2007); (A2). Le point de fusion du chlorure de 4-formylbenzoyle synthétisé est conforme aux données décrites dans les références suivantes : Graffner-Nordberg, Malin; Sjoedin, Karin; Tunek, Anders; Hallberg, Anders Chemical & Pharmaceutical Bulletin, 1998 , vol.46, 4, p.591 - 601 *et* Kuhlmann; Alexander Inorganica Chimica Acta, 1979 , vol.34, p.197,207 *et* Simonis Chemische Berichte, 1912 , vol.45, p.1586

### b) Préparation du 4-formyl-N-[2-(2-oxoimidazolidin-1-yl)ethyl]benzamide

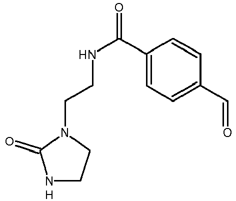

A une suspension de 1-(2-aminoethyl)imidazolidin-2-one (12,6 g, 0,098 mol) et Et₃N (19,8 g, 0,195 mol) dans le THF sec (300 mL) à -35°C, est ajoutée une solution du chlorure de 4-formylbenzoyle (16.5 g, 0,098 mol) dans le THF sec (100 mL) en 30 minutes. Pendant l'addition, la température du milieu réactionnel est maintenue entre - 35 et - 38°C. La température du milieu réactionnel est ensuite ramenée lentement à température ambiante sur 4 heures. Le précipité obtenu (principalement le produit attendu en mélange avec le chlorhydrate de la triéthylamine Et₃N•HCl) est filtré et lavé par le THF (2 fois par 20 mL). Le brut réactionnel est solubilisé dans une solution aqueuse de Na₂CO₃ (3,4 g, 0,032 mol dans 40 mL d'eau). Le composé attendu est extrait plusieurs fois par EtOAc (volume total : 3,5 L). Les phases organiques rassemblées sont séchées sur Na₂SO₄ et concentrées sous pression réduite (T_{bain} = 40°C).

Un solide (5,53 g, rendement 22 %) de point de fusion 138 °C est obtenu.

La pureté molaire est supérieure à 81 % (RMN ¹H). Ce composé est directement engagé dans l'étape suivante sans purification supplémentaire.

### Caractérisation RMN ¹H et ¹³C

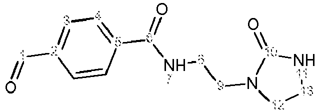

**Tableau 11**

| **N° atomes** | **δ du ¹H (ppm)** | **δ du** ¹³**C (ppm)** |
|---|---|---|
| 1 | 10,01 | 192,6 |
| 2 | / | 137,4 |
| 3/4 | 7,93 | 128,2 + 129,7 |
| 5 | / | 139,3 |
| 6 | / | 165,2 |
| 7 | 8,69 | / |
| 8 | 3,33 | 38,2 |
| 9 | 3,18 | 42,9 |
| 10 | / | 162,0 |
| 11 | 6,24 | / |
| 12 | 3,35 | 45,0 |
| 13 | 3,15 | 37,9 |

Solvant utilisé : DMSO - Calibration sur le signal du DMSO à 2.44 ppm en ¹H, 39.5 ppm en ¹³C.

### c) Préparation de la N-phénylhydroxylamine

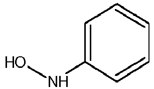

La synthèse de ce composé à partir du nitrobenzène est décrite dans Organic Syntheses, Coll. Vol. 1. p. 445 (1941*) ;* Vol . 4 . p. 57 (1925*).*

### d) Préparation de la (Z,E)-N-(4-(2-(2-oxoimidazolidin-1-yl)ethylcarbamoyl)benzylidene)aniline oxide

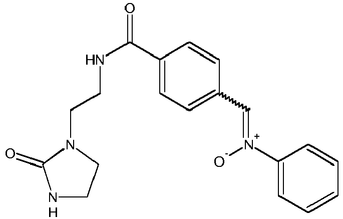

A une solution de 4-formyl-N-[2-(2-oxoimidazolidin-1-yl)ethyl]benzamide (5,3 g, 0,020 mol) dans EtOH (50 mL) est ajoutée une solution de *N*-phenylhydroxylamine (2,21 g, 0,020 mol) dans EtOH (10 mL). Le mélange réactionnel est porté au reflux pendant 4 heures puis refroidi jusqu'à température ambiante. Le précipité obtenu est filtré, lavé par EtOH (3 fois par 5 mL) et séché à température ambiante sous air.

Un solide blanc (4,65 g, rendement 66 %) de point de fusion 209 °C est obtenu.

La pureté molaire est supérieure à 92 % (RMN ¹H).

### Caractérisation RMN ¹H et ¹³C

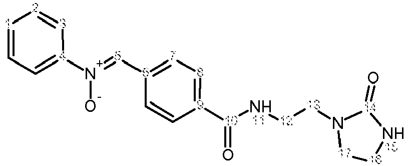

**Tableau 12**

| **N° atomes** | **δ du** ¹**H (ppm)** | **δ du** ¹³**C (ppm)** |
|---|---|---|
| 1/2 | 7,50 | 128,9 + 129,9 |
| 3 | 7,87 | 121,3 |
| 4 | / | 148,1 |
| 5 | 8,53 | 132,7 |
| 6 | / | 133 |
| 7 | 8,47 | 128,2 |
| 8 | 7,87 | 126,9 |
| 9 | / | 135,3 |
| 10 | / | 165,3 |
| 11 | 8,58 | / |
| 12 | 3,33 | 37,5 |
| 13 | 3,18 | 42,4 |
| 14 | / | 162,1 |
| 15 | 6.25 | / |
| 16 | 3,16 | 37,2 |
| 17 | 3,36 | 44,5 |

Solvant utilisé : DMSO - Calibration sur le signal du DMSO à 2.44 ppm en ¹H, 39.5 ppm en ¹³C.

### Caractérisation par spectrométrie de masse

**C**₁₉**H**₂₀**N**₄**O**₃, **Mw= 352.38 g/mol**

L'échantillon a été analysé par introduction directe à la spectrométrie de masse en utilisant l'électrospray comme mode d'ionisation (ID/ESI).

### Préparation de l'échantillon :

Environ 20 mg d'échantillon a été mis en solution dans 0.5 ml de DMSO + 24.5 ml de méthanol, puis dilué au 1/100 dans du méthanol pour l'analyse par ID/ESI.

### Mode positif

**m/z :** 375 ([M+Na]⁺), 727 ([2M+Na]⁺)

### Mode négatif

**m/z :** 351 ([M-H]⁻), 703 ([2M-H]⁻)

## Revendications

1. Composé comprenant au moins un groupement Q, et au moins un groupement A reliés entre eux par au moins un groupement « espaceur » Sp dans lequel :
- Q comprend une fonction oxyde de nitrile, nitrone, ou nitrile imine, répondant à l'une des formules (VII), (VIII) ou (IX) suivantes : 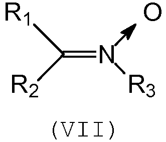 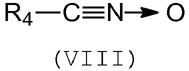 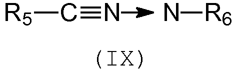 dans lesquelles R1 à R6 sont choisis indépendamment parmi un groupe espaceur Sp, un atome d'hydrogène, un groupe alkyle en C1-C20 linéaire ou ramifié, un groupe cycloalkyle en C3-C20 linéaire ou ramifié, un groupe aryle en C6-C20 linéaire ou ramifié, et un groupe de formule (X) 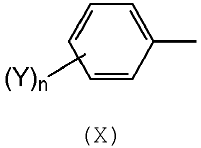 dans laquelle n représente 1, 2, 3, 4 ou 5 et chaque Y représente indépendamment un groupe espaceur Sp, un groupe alkyle ou un halogène
- A comprend un groupe associatif comprenant au moins un atome d'azote, répondant à la formule (II) suivante : 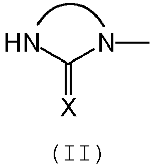 où X désigne un atome d'oxygène ou de soufre,
- Sp est une chaîne alkyle linéaire ou ramifiée en C1-C24, comprenant éventuellement un ou plusieurs hétéroatomes choisis parmi les atomes d'azote ou d'oxygène.

2. Composé selon la revendication 1 **caractérisé en ce que** X dans la formule (II) désigne un atome d'oxygène.

3. Composé selon la revendication 1 ou 2 **caractérisé en ce que** R1 à R6 sont choisis indépendamment parmi un groupe de formule (X) 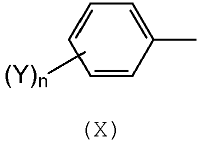 dans laquelle n représente 1, 2, 3, 4 ou 5 et chaque Y représente indépendamment un groupe espaceur Sp, un groupe alkyle ou un halogénure.

4. Composé selon l'une quelconque des revendications 1 à 3 **caractérisé en ce que** le groupement « espaceur » est une chaîne alkyle linéaire en C1-C6 comprenant éventuellement un ou plusieurs hétéroatomes choisis parmi les atomes d'azote ou d'oxygène.

5. Composé selon l'une quelconque des revendications 1 à 4 **caractérisé en ce que** le groupement Q est un groupement de formule (XI): 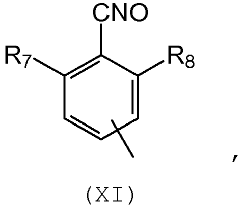 dans laquelle R7 et R8 représentent indépendamment un hydrogène ou un groupe alkyle en C1-C5, un alcoxyle ou un halogénure et le groupement A est un groupement de formule (XII)

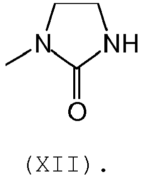

6. Composé selon la revendication 5 **caractérisé en ce que** R7 et R8 représentent indépendamment un groupe méthyle ou un atome de chlore.

7. Composé selon l'une quelconque des revendications 1 à 6 **caractérisé en ce qu'**il est choisi parmi les composés de formule (XIII) à (XXI) suivantes:

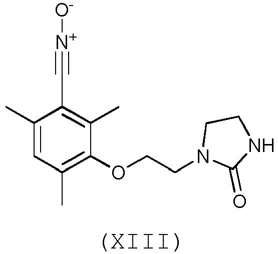

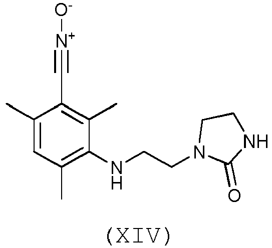

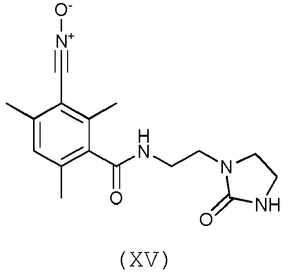

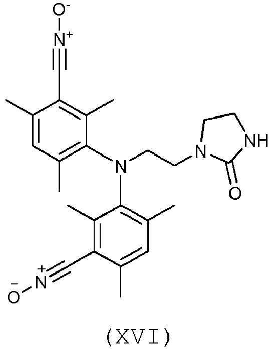

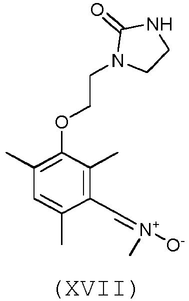

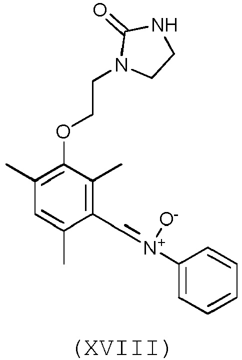

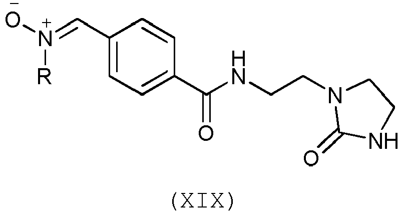

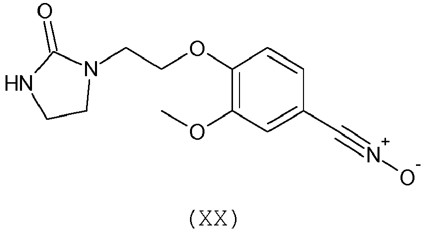

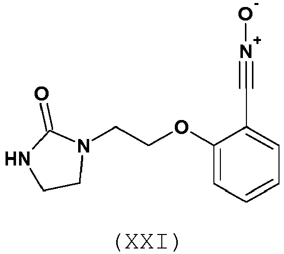

8. Composé selon l'une quelconque des revendications 1 à 6 **caractérisé en ce qu'** il est choisi parmi les composés de formule (XXII) et (XXIII) suivantes: 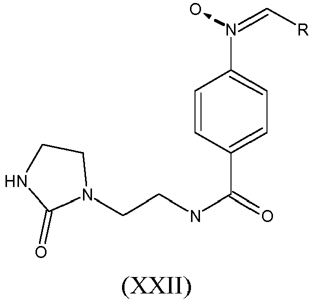 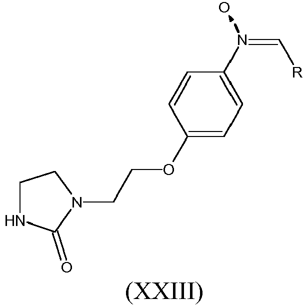 dans lesquelles R répond à la définition donnée pour R1 à R6.

## Patentansprüche

1. Verbindung, umfassend mindestens eine Gruppierung Q sowie mindestens eine Gruppierung A, verknüpft miteinander durch mindestens eine "Spacer"-Gruppierung Sp, in der:
- Q eine Nitriloxid-, Nitron- oder Nitriliminfunktion umfasst, entsprechend einer der folgenden Formeln (VII), (VIII) oder (IX) : 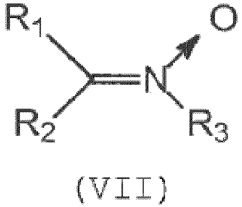 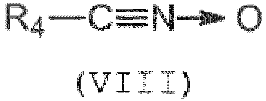 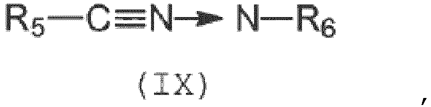 worin R1 bis R6 unabhängig aus einer Spacer-Gruppe Sp, einem Wasserstoffatom, einer geradkettigen oder verzweigten C1-C20-Alkylgruppe, einer geradkettigen oder verzweigten C3-C20-Cycloalkylgruppe, einer geradkettigen oder verzweigten C6-C20-Arylgruppe und einer Gruppe der Formel (X) 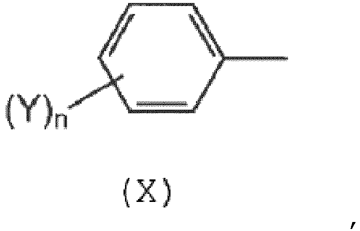 worin n 1, 2, 3, 4 oder 5 bedeutet und jedes Y unabhängig eine Spacer-Gruppe Sp, eine Alkylgruppe oder ein Halogen bedeutet, ausgewählt sind,
- A eine assoziative Gruppe umfasst, die mindestens ein Stickstoffatom umfasst, entsprechend der folgenden Formel (II): 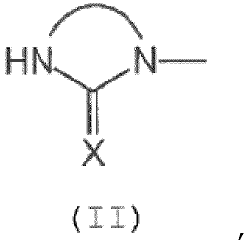 wo X ein Sauerstoff- oder Schwefelatom bedeutet,
- Sp eine geradkettige oder verzweigte C1-C24-Alkylkette ist, die gegebenenfalls ein oder mehrere Heteroatome, ausgewählt aus Stickstoff- oder Sauerstoffatomen, umfasst.

2. Verbindung nach Anspruch 1, **dadurch gekennzeichnet, dass** X in Formel (II) ein Sauerstoffatom bedeutet.

3. Verbindung nach Anspruch 1 oder 2, **dadurch gekennzeichnet, dass** R1 bis R6 unabhängig aus einer Gruppe der Formel (X) 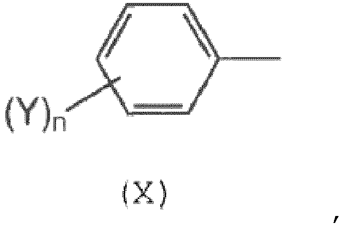 worin n 1, 2, 3, 4 oder 5 bedeutet und jedes Y unabhängig eine Spacer-Gruppe Sp, eine Alkylgruppe oder ein Halogenid bedeutet, ausgewählt sind.

4. Verbindung nach einem der Ansprüche 1 bis 3, **dadurch gekennzeichnet, dass** die "Spacer"-Gruppierung eine geradkettige C1-C6-Alkylkette ist, die gegebenenfalls ein oder mehrere Heteroatome, ausgewählt aus Stickstoff- oder Sauerstoffatomen, umfasst.

5. Verbindung nach einem der Ansprüche 1 bis 4, **dadurch gekennzeichnet, dass** es sich bei der Gruppierung Q um eine Gruppierung der Formel (XI) handelt: 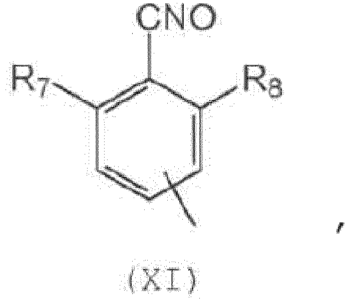 worin R7 und R8 unabhängig Wasserstoff oder eine C1-C5-Alkylgruppe, eine Alkoxylgruppe oder ein Halogenid bedeuten und die Gruppierung A eine Gruppierung der Formel (XII) ist:

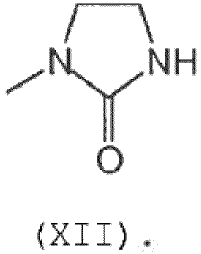

6. Verbindung nach Anspruch 5, **dadurch gekennzeichnet, dass** R7 und R8 unabhängig eine Methylgruppe oder ein Chloratom bedeuten.

7. Verbindung nach einem der Ansprüche 1 bis 6, **dadurch gekennzeichnet, dass** sie unter den folgenden Verbindungen der Formeln (XIII) bis (XXI) ausgewählt ist:

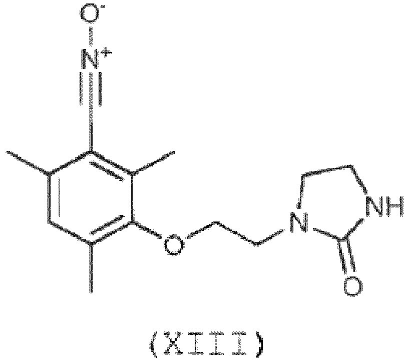

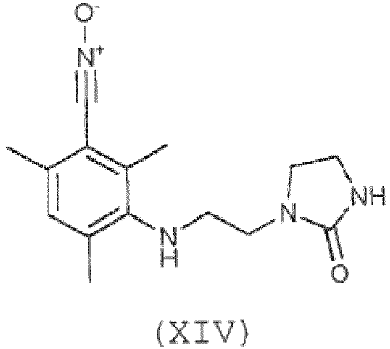

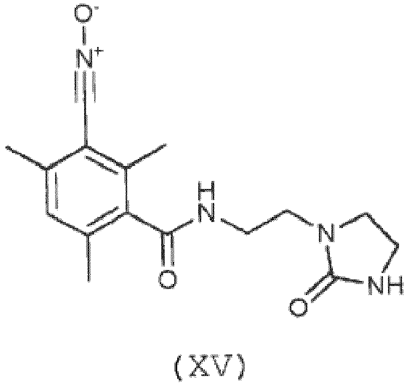

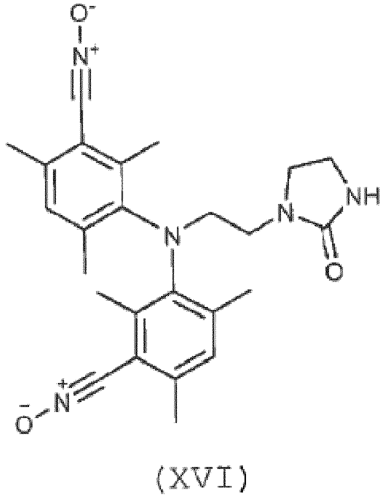

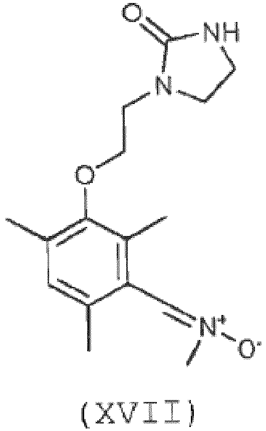

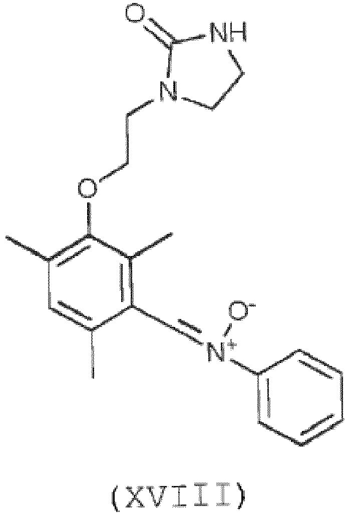

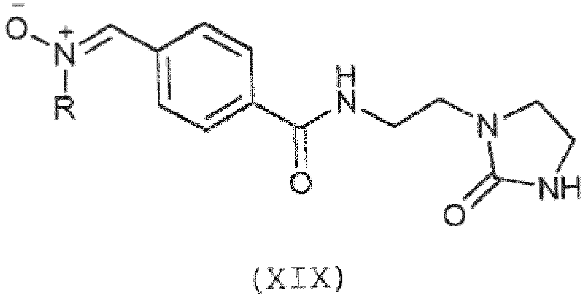

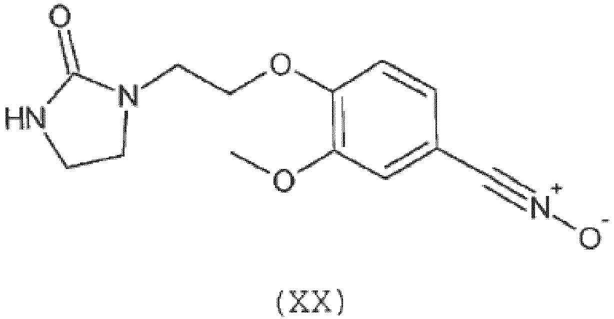

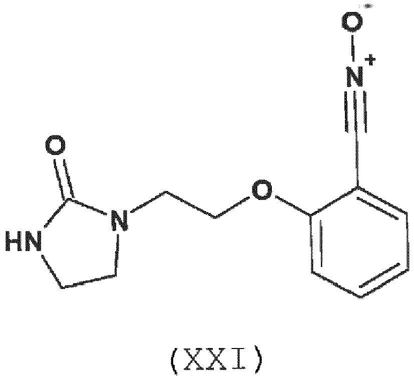

8. Verbindung nach einem der Ansprüche 1 bis 6, **dadurch gekennzeichnet, dass** sie unter den folgenden Verbindungen der Formeln (XXII) und (XXIII) ausgewählt ist: 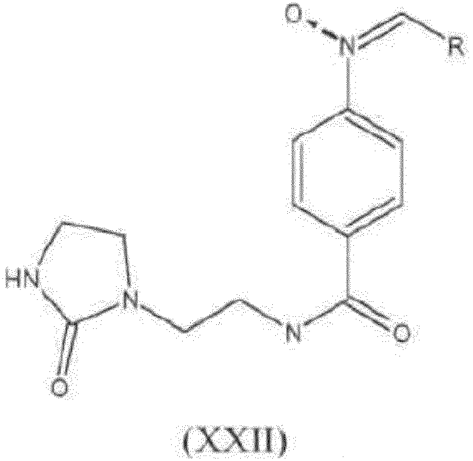 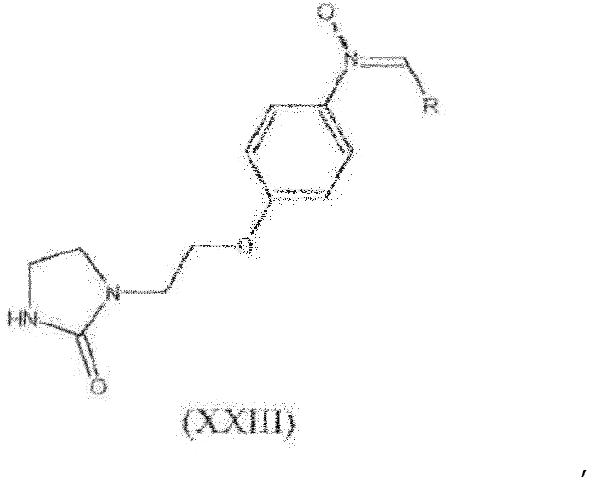 worin R der für R1 bis R6 angeführten Definition entspricht.

## Claims

1. Compound comprising at least one group Q and at least one group A connected to one another by at least one "spacer" group Sp in which:
- Q comprises a nitrile oxide, nitrone or nitrile imine function, corresponding to one of the following formulae (VII), (VIII) and (IX) : 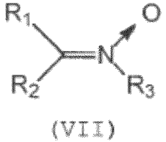 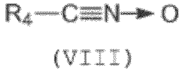 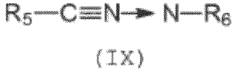 in which R₁ to R₆ are chosen independently from a spacer group Sp, a hydrogen atom, a linear or branched C₁-C₂₀ alkyl group, a linear or branched C₃-C₂₀ cycloalkyl group, a linear or branched C₆-C₂₀ aryl group, and a group of formula (X) 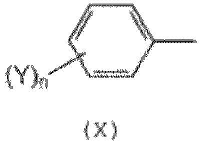 in which n represents 1, 2, 3, 4 or 5 and each Y independently represents a spacer group Sp, an alkyl group or a halogen,
- A comprises an associative group comprising at least one nitrogen atom, corresponding to formula (II) below: 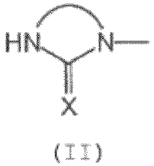 where X denotes an oxygen or sulphur atom,
- Sp is a linear or branched C₁-C₂₄ alkyl chain, optionally comprising one or more heteroatoms chosen from nitrogen or oxygen atoms.

2. Compound according to Claim 1, **characterized in that** X in formula (II) denotes an oxygen atom.

3. Compound according to Claim 1 or 2, **characterized in that** R₁ to R₆ are chosen independently from a group of formula (X) 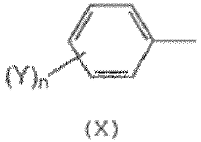 in which n represents 1, 2, 3, 4 or 5 and each Y independently represents a spacer group Sp, an alkyl group or a halide.

4. Compound according to any one of Claims 1 to 3, **characterized in that** the "spacer" group is a linear C₁-C₆ alkyl chain optionally comprising one or more heteroatoms chosen from nitrogen or oxygen atoms.

5. Compound according to any one of Claims 1 to 4, **characterized in that** the group Q is a group of formula (XI): 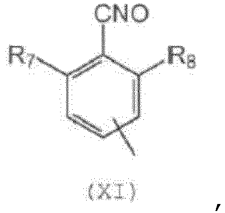 in which R₇ and R₈ independently represent a hydrogen or a C₁-C₅ alkyl group, an alkoxyl or a halide and the group A is a group of formula (XII)

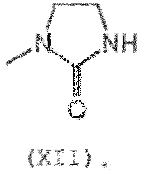

6. Compound according to Claim 5, **characterized in that** R₇ and R₈ independently represent a methyl group or a chlorine atom.

7. Compound according to any one of Claims 1 to 6, **characterized in that** it is chosen from the compounds of formulae (XIII) to (XXI) below:

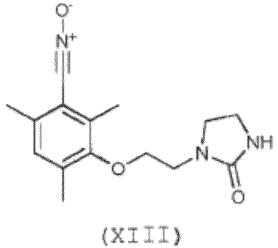

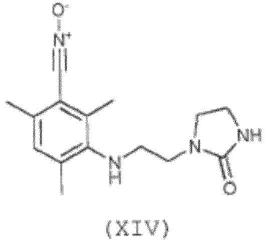

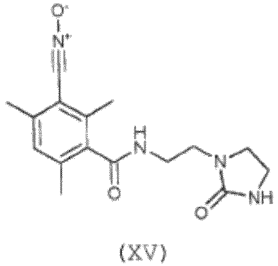

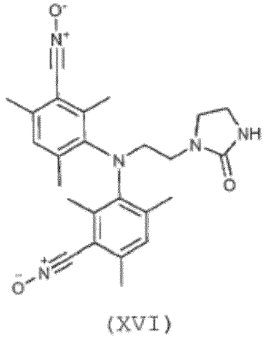

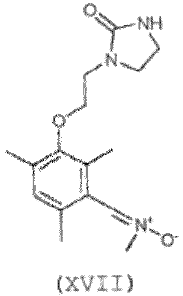

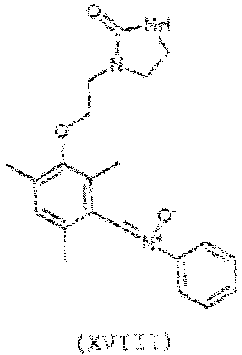

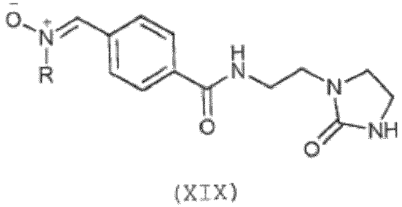

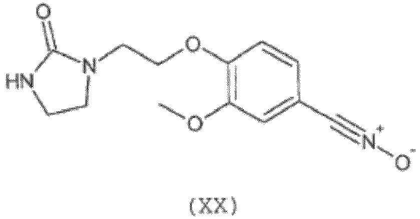

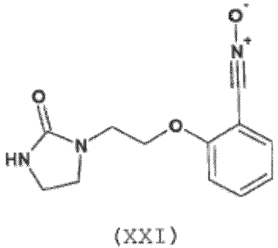

8. Compound according to any one of Claims 1 to 6, **characterized in that** it is chosen from the compounds of formulae (XXII) and (XXIII) below: 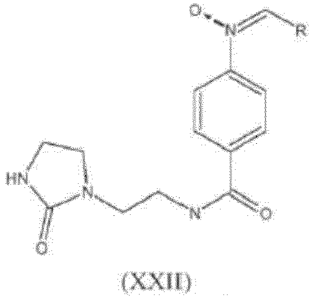 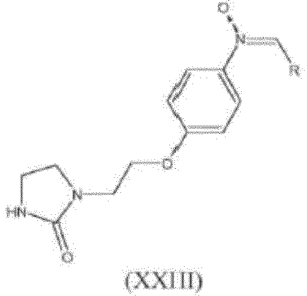 in which R corresponds to the definition given for R₁ to R₆.
